(19) Europäisches Patentamt
European Patent Office
Office européen des brevets

(11) **EP 2 925 225 B1**

(12) **EUROPÄISCHE PATENTSCHRIFT**

(45) Veröffentlichungstag und Bekanntmachung des Hinweises auf die Patenterteilung:
**09.01.2019   Patentblatt 2019/02**

(21) Anmeldenummer: **13750560.8**

(22) Anmeldetag: **16.08.2013**

(51) Int Cl.:
***A61B 5/107*** *(2006.01)*

(86) Internationale Anmeldenummer:
**PCT/EP2013/067144**

(87) Internationale Veröffentlichungsnummer:
**WO 2014/082763 (05.06.2014 Gazette 2014/23)**

(54) **LÄNGENMESSGERÄT**

LENGTH MEASURING DEVICE

APPAREIL DE MESURE DE LONGUEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.11.2012   DE 102012220412**

(43) Veröffentlichungstag der Anmeldung:
**07.10.2015   Patentblatt 2015/41**

(73) Patentinhaber: **seca ag**
**4153 Reinach BL 1 (CH)**

(72) Erfinder:
• **WJATSCHESLAW, Galjan**
**24568 Nützen (DE)**

• **SCHMIDT, Jan**
**22941 Bargteheide (DE)**
• **NESSLER, Andreas**
**22147 Hamburg (DE)**
• **KRAUSE, Bernd**
**21435 Stelle (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**WO-A1-85/01800     DE-A1- 3 428 132**
**JP-A- S5 882 110     JP-A- H02 239 843**

EP 2 925 225 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Längenmessgerät zur Messung der Körperlänge einer Person mit einem Messschieber, einer linearen Führung in Form eines Hohlprofils, an dem der Messschieber außen verschiebbar gelagert ist, um ihn in Auflage auf den Kopf der zu messenden Person bringen zu können, einem innerer Schieber, der im Inneren des Hohlprofils verschiebbar gelagert ist und mit dem Messschieber gekoppelt ist, so dass der innere Schieber jeder Bewegung des Messschiebers entlang des Hohlprofils folgt, einer Messeinrichtung zur Messung der Position des inneren Schiebers entlang des Hohlprofils und einer im Außenraum des Hohlprofils sichtbaren Anzeige der durch die Messeinrichtung ermittelten Länge nach Maßgabe der gemessen Position des inneren Schiebers.

Die Erfindung ist auf Längenmessgeräte zur Messung der Körpergröße von Personen gerichtet. Solche Längenmessgeräte werden auch als Stadiometer bezeichnet. Ein typisches derartiges Längenmessgerät weist einen Messstab auf, der als vertikale lineare Führung für einen Messschieber (Kopfschieber) ausgebildet ist. Der Messstab ist vertikal ausgerichtet und an einer Wand montiert oder an einer Plattform befestigt. Zum Messen der Körpergröße tritt die Person vor den Messstab, wonach der Kopfschieber an dem Messstab heruntergeschoben wird, bis er in Auflage auf den Kopf der zu messenden Person kommt. An dem Messstab ist eine Messskala mit Skaleneinteilungsstrichen vorgesehen. In dem Messschieber ist wiederum eine Leseeinheit vorhanden, die die Skaleneinteilungsstriche beim Verschieben des Messschiebers an dem Messstab registriert und somit die inkrementelle Änderung der Position des Messschiebers erfasst. In den Skaleneinteilungsstrichen kann auch die absolute Höhe eines Skaleneinteilungsstrichs kodiert sein, so dass mit der Leseeinheit die Höhe des Kopfschiebers bestimmt werden kann, die dann auf einer Anzeige an dem Messschieber angezeigt wird.

Ein anderer Typ eines Längenmessgerätes für die Körpergröße weist ein Winkelstück auf, das von einer die Messung der Körperlänge durchführenden Person gehalten wird. Ein Schenkel des Winkelstücks wird in Auflage auf den Kopf der zu messenden Person gehalten. Von diesem Schenkel in Auflage auf dem Kopf steht ein zweiter Schenkel senkrecht ab, wobei das Winkelstück so gehalten wird, dass der zweite Schenkel vertikal auf den Boden gerichtet verläuft. In dem zweiten Schenkel ist eine Abstandsmesseinrichtung mit einem Ultraschallwandler vorgesehen, die aus der Laufzeit eines ausgesendeten Ultraschallsignals, das am Boden reflektiert wird und zum Ultraschallwandler zurückläuft, die Höhe des in Auflage auf dem Kopf der zu messenden Person befindlichen ersten Schenkels über dem Boden und damit die Körpergröße bestimmt und anzeigt. Ein Nachteil dieses Typs von Längenmessgerät besteht darin, dass Messungenauigkeiten dadurch auftreten können, dass die die Messung durchführende Person das Winkelstück nicht exakt so ausgerichtet hält, dass der zweite Schenkel genau vertikal auf den Boden gerichtet ist. Weiterhin ist nachteilig, dass wechselnde Umgebungsbedingungen (z.B. Staub oder anderer Verunreinigungen in der Luft) oder auf dem Boden liegende Gegenstände die Messung verfälschen können.

Ein weiterer Typ eines Längenmessgerätes weist eine Plattform, auf die die zu messende Person tritt, und einen vertikal oberhalb der zu messenden Personen fest aufgehängten horizontalen Träger auf. An dem Träger ist eine Abstandsmesseinrichtung auf Basis von Ultraschallwellenlaufzeit angebracht, die auf den Kopf der auf der Plattform stehenden, zu messenden Person gerichtet ist. Die zu messende Person trägt eine Kappe, um eine wohldefinierte Reflexion der Ultraschallwellen von der Oberseite des Kopfes zu gewährleisten. Aus dem Abstand der Oberseite des Kopfes der Person zu der horizontal oberhalb der zu messenden Person fest installierten Abstandsmesseinrichtung lässt sich aus der Differenz der Aufhängungshöhe der Abstandsmesseinrichtung und dem gemessenen Abstand zur Oberseite des Kopfes die Körperlänge der zu messenden Person ableiten. Auch dieses Längenmessgerät ist fehleranfällig, da die Messung durch wechselnde Umgebungsbedingungen und störende Einflüsse in der offenen Messstrecke zwischen dem Ultraschallwandler und der Oberseite des Kopfes der zu messenden Person verfälscht werden kann.

Aus WO 98/17974 A1 ist ein Längenmessgerät bekannt, das nicht als Längenmessgerät für Personen ausgebildet ist. Vielmehr soll die Position eines Messschiebers verfolgt werden. Das Längemessgerät hat eine lineare Führung in Form eines hohlen Gehäuses, an dem der Messschieber außen verschiebbar gelagert ist. Im Inneren des Gehäuses ist ein innerer Schieber darin verschiebbar gelagert. Eine Magnetanordnung koppelt den Messschieber und den inneren Schieber magnetisch, so dass der innere Schieber jeder Bewegung des Messschiebers entlang der Führung folgt. Der innere Schieber ist in Schleifkontakt mit einem linearen Potentiometer, um ein Spannungssignal bereitzustellen, das proportional zu einer Position des inneren Schiebers ist. Aus dem Potentiometersignal wird die Position des inneren Schiebers und damit die des damit gekoppelten Messschiebers entlang der linearen Führung abgeleitet. Durch die Messung der Position des inneren Schiebers im Inneren des Gehäuses sind zwar gewissen Störfaktoren wie Rauch oder Staub, die nicht in das Innere des Gehäuses eindringen, in ihrem Einfluss auf die Messgenauigkeit reduziert. Das Potentiometer weist Schleifkontakte auf, die Abrieb erzeugen und dadurch mit der Zeit verschleißen. Um dem entgegen zu wirken, müssen hochwertige Materialien eingesetzt werden, was natürlich die Herstellungskosten erhöht. Aber auch unter diesen Bedingungen kann der Verschleiß zu einer Verschlechterung der Messgenauigkeit führen.

Aus DE 34 28 132 A1 ist ein Längenmessgerät zur Messung der Füllstandshöhe in einem Behälter bekannt. Ein Schwimmkörper ist gleitfähig an einem Rohr gelagert und magnetisch mit einem Teil im Inneren des Rohrs gekoppelt, so dass das Teil der vertikalen Position des Schwimmkörpers folgt. Die Messung des Abstands zu diesem Teil im Inneren des Rohrs erfolgt durch Ultraschalllaufzeitmessung.

Aus JP H02 239843 A ist ein Längenmessgerät zur Messung der Körperlänge einer Person mit den Merkmalen des Oberbegriffs von Patentanspruch 1 bekannt. Das Längenmessgerät hat ein vertikales Hohlprofil, an dem ein Messschieber in seiner Höhe verschoben werden kann. Der Messschieber ist mechanisch mit einer Reflexionsplatte im Inneren des Hohlprofils verbunden. Am Boden des Hohlprofils ist eine Laseranordnung vorgesehen, die einen Laserstrahl auf die Reflexionsplatte richten und den reflektierten Strahl erfassen kann, woraus der Abstand der Reflexionsplatte von der Laseranordnung bestimmt werden kann. Auf diese Weise wird die Position des Messschieber aus und damit die Körperlänge der zu messenden Person bestimmt. Aufgrund der mechanischen Kopplung zwischen Messschieber und Reflexionsplatte kann das Hohlprofil nicht vollständig geschlossen sein, da die mechanische Verbindung zwischen Messschieber und Reflexionsplatte vom Äußeren in das Innere des Hohlprofils verlaufen muss.

Es ist Aufgabe der vorliegenden Erfindung, ein Längenmessgerät so auszubilden, dass es unabhängig von veränderlichen Umgebungsbedingungen eine hohe Messgenauigkeit bietet, insbesondere die Möglichkeit bietet, Referenzmessungen zur Kalibration der Längenmessung durchzuführen.

Zur Lösung dieser Ausgabe dient ein Längenmessgerät mit den Merkmalen des Patentanspruchs 1. Vorteilhafte Ausführungsformen der Erfindung sind in den Unteransprüchen aufgeführt.

Erfindungsgemäß ist vorgesehen, dass eine Magnetanordnung den Messschieber und den inneren Schieber magnetisch koppelt. Ferner ist vorgesehen, dass die Messeinrichtung eine Schallquelle und einen Schallempfänger im Inneren des Hohlprofils sowie eine damit verbundene Steuer- und Auswerteeinheit aufweist. Die Steuer- und Auswerteeinheit ist dazu eingerichtet, die Schallquelle zur Aussendung eines Schallsignals anzuregen und Ausgabesignale des Schallempfängers auszuwerten, um aus einem an dem inneren Schieber reflektierten Signal dessen Laufzeit zu bestimmen und daraus die Position des inneren Schiebers entlang der linearen Führung zu berechnen. Das Hohlprofil und/oder der innere Schieber sind so ausgestaltet, dass ein weiteres, an einem anderen bekannten Punkt erzeugtes Reflexionssignal auftritt. Die Steuer- und Auswerteeinheit ist weiter dazu eingerichtet, das weitere reflektierte Signal zu erfassen und dessen Laufzeit als Referenzmessung in die Berechnung der Position des inneren Schiebers eingehen zu lassen.

Auf diese Weise kann die Position des Messschiebers anhand der Position des inneren Messschiebers unabhängig von sich verändernden atmosphärischen Umgebungsbedingungen, wie Staub in der Luft und dergleichen, durchgeführt werden, da die Messung im Inneren des Hohlprofils demgegenüber weitgehend abgeschirmt ist. Ferner kann durch die Messung eines weiteren reflektierten Signals, bei bekannter Position des Reflexionspunktes des weiteren reflektierten Signals oder bei bekanntem Abstand zum Reflexionsort des ersten an dem inneren Schieber reflektierten Signals eine Kalibration der Berechnung der Position des inneren Schiebers durchgeführt werden, so dass auch veränderliche Umgebungsbedingungen wie die Umgebungstemperatur und Luftfeuchtigkeit kompensiert werden können.

Eine Referenzmessung muss natürlich nicht bei jeder Bestimmung der Position des inneren Schiebers durchgeführt werden. Es genügt, wenn eine Referenzmessung zur erneuten Kalibration gelegentlich wiederholt wird.

In einer vorteilhaften Ausführungsform ist die Schallquelle an einem Ende des Hohlprofils angeordnet. Ferner weist der innere Schieber einen ringförmigen Aufbau mit einer Durchgangsöffnung auf. Bei einem solchen Aufbau wird zunächst ein Schallsignal an dem unteren Wandbereich des ringförmigen Schiebers reflektiert. Einen weiteren Reflexionspunkt stellt der obere Rand der Durchgangsöffnung, der von der Schallquelle abgewandt ist, dar, da auch von diesem Rand Schallwellen zurück zum Schallempfänger reflektiert werden. Bei bekannter axialer Länge der Durchgangsöffnung durch den inneren Schieber lässt sich so aus der Differenz der Laufzeitmessungen der beiden reflektierten Signale eine absolute Kalibration der Längenberechnung durchführen. Um die Bildung des Weiteren reflektierten Signals am oberen Rand der Durchgangsöffnung des inneren Schiebers zu verstärken, kann am oberen Rand beispielsweise ein in die Öffnung der Durchgangsöffnung hineinragender Überstand vorgesehen sein, an dem ein Teil eines die Durchgangsöffnung des inneren Schiebers passierenden Ultraschallsignals reflektiert wird.

Da der Abstand des weiteren Reflexionsortes (hier die axiale Länge des inneren Schiebers) bekannt ist, kann die Steuer- und Auswerteeinheit nach Erfassen des ersten reflektierten Schallsignals in einem bestimmten Zeitfenster nach dem weiteren reflektierten Schallsignal suchen, da der ungefähre Abstand zwischen den beiden reflektierten Signalen bekannt ist (bis auf Schwankungen durch veränderliche Umgebungstemperatur und Luftfeuchtigkeit).

In einer weiteren vorteilhaften Ausführungsform hat der innere Schieber wiederum einen ringförmigen Aufbau mit einer Durchgangsöffnung. Die Steuer- und Auswerteeinheit ist dazu angepasst, ein an dem inneren Schieber reflektiertes Schallsignal zu erfassen und dessen Laufzeit zu bestimmen. Darüber hinaus ist das der Schallquelle gegenüberliegende Ende des Hohlprofils mit einer Wand verschlossen. Die Steuer- und Auswerteeinheit ist weiter dazu eingerichtet, ein Schallsignal, das die Durchgangsöffnung des inneren Schiebers durchlaufen hat und an der Wand des gegenüberliegenden Endes des Hohlprofils reflektiert worden ist, zu erfassen und aus dessen Laufzeit bei bekannter Länge des Hohlprofils eine Kalibration für die Berechnung der Position des inneren Schiebers aus der Laufzeitmessung abzuleiten.

In einer bevorzugten Ausführungsform können auch an der Innenwand des Hohlprofils an vorbestimmten Positionen Störstellen angeordnet sein, die reflektierte Schallsignale erzeugen, die als weitere reflektierte Schallsignale erfasst werden können, um damit weitere Laufzeiten für bekannte Reflexionspunkte entlang des Hohlprofils zu bestimmen.

In einer bevorzugten Ausführungsform ist das Hohlprofil des Längenmessgerätes vollkommen geschlossen, so dass veränderliche atmosphärische Umgebungsbedingungen keinen Einfluss auf die Messung im Inneren des dem gegenüber abgeschirmten Hohlprofils haben.

Vorzugsweise ist die Steuer- und Auswerteeinheit dazu eingerichtet, die Entfernungsbestimmung über ein TOF-Verfahren (Time of Flight) durchzuführen. Derartige Verfahren sind im Stand der Technik bekannt, wozu beispielhaft auf die Artikel "A high accuracy ultrasonic distance measurement system using binary frequency shift-keyed signal and phase detection", von S. S. Huang et al., Review of Scientific Instruments, Vol. 73, Nr. 10, Oktober 2002, Seiten 3671 - 3677, "A new method for high resolution ultrasonic ranging in air" von R. Queiros et al., XVIII Imeko World Congress, Metrology for a Sustainable Development, September 17 - 22, 2006, Rio de Janeiro, Brasilien, und "Robust High-Accuracy Ultrasonic Range Measurement System" von M. M. Saad et al., IEEE Transactions on Instrumentation and Measurement, Vol. 60, Nr. 10, Oktober 2011, Seiten 3334 - 3341 verwiesen wird.

Zur Durchführung eines TOF-Verfahrens kann die Steuer- und Auswerteeinheit auch dazu eingerichtet sein, die Schallquelle mit einem periodischen Anregungssignal anzuregen, dem eine charakteristische Signaleigenschaft wie ein Phasensprung, ein Amplitudensprung oder ein Frequenzsprung aufgeprägt ist, und durch Bilden einer Korrelation der vom Schallempfänger aufgenommenen reflektierten Signale mit dem Anregungssignal die Laufzeit zu berechnen. Insbesondere kann zwischen den genannten Signalen eine Kreuzkorrelation berechnet werden und die Laufzeit beim Maximum der Kreuzkorrelation bestimmt werden.

In einer bevorzugten Ausführungsform weist die Magnetanordnung wenigstens einen Permanentmagneten an dem Messschieber und einen Permanentmagneten an dem inneren Schieber auf, die so angeordnet sind, dass entgegengesetzt Pole der beiden Permanentmagnete aufeinander zu weisend zueinander ausgerichtet sind. Vorzugsweise sind jeweils vier Permanentmagnete an dem inneren Schieber und an dem Messschieber so zueinander angeordnet, dass jeweils ein Paar eines Permanentmagneten an dem Messschieber und an dem inneren Schieber mit entgegengesetzten Polen aufeinander zu weisend zueinander ausgerichtet sind. Alternativ weist die Magnetanordnung nur einen Permanentmagneten an einem von Messschieber und innerem Schieber auf, wobei dann die andere Komponente von Messschieber und innerem Schieber ferro- oder paramagnetisches Material enthält, so dass Messschieber und innerer Schieber magnetisch gekoppelt sind.

Die äußeren Abmessungen des inneren Schiebers sind vorzugsweise an die inneren Abmessungen des Hohlprofils angepasst, so dass der innere Schieber ohne Spiel, aber gleitfähig in dem Hohlprofil sitzt. Entsprechend sind die inneren Abmessungen des Messschiebers so an die äußeren Abmessungen des Hohlprofils angepasst, dass der Messschieber ohne Spiel, aber gleitfähig außen auf dem Hohlprofil gelagert ist.

Vorzugsweise ist der Innendurchmesser des Hohlprofils so gewählt und sind die Schallquelle und die Steuer- und Auswerteeinheit so eingerichtet, dass der Innendurchmesser des Hohlprofils kleiner als die halbe Wellenlänge der Schallwellen des Schallsignals ist. Damit wird gewährleistet, dass sich der Schall im Hohlprofil als ebene Welle ausbreitet. Bei kürzeren Wellenlängen könnte der Schall sich auch in anderen Moden ausbreiten, d.h. er würde an den Wänden reflektiert werden und damit einen längeren Weg zurücklegen. Effektiv haben diese Moden eine geringere Schallgeschwindigkeit und überlagern das Signal der ebenen Welle leicht zeitversetzt. Grundsätzlich sollte die Frequenz möglichst hoch gewählt werden, um eine hohe Zeitauflösung zu erhalten. Die genannten Bedingung lässt sich insbesondere im Fall der Verwendung von Schallsignalen mit akustischen Schallwellenlängen gut realisieren (unter akustischen Schallsignalen werden hier und im Folgenden Signale mit Schallwellenlängen im vom menschlichen Ohr hörbaren Bereich bezeichnet).

In einer bevorzugten Ausführungsform ist die Schallquelle eine Ultraschallquelle und der Schallempfänger ein Ultraschallempfänger. Insbesondere können in diesem Fall die Ultraschallquelle und der Ultraschallempfänger durch einen einheitlichen Ultraschallwandler in Form eines Transceivers gebildet sein.

In einer alternativen Ausführungsform kann die Schallquelle einen Lautsprecher zur Erzeugung eines akustischen Schallsignals und der Schallempfänger ein Mikrophon zum Aufnehmen akustischer Schallsignale aufweisen.

Die Erfindung wird im Folgenden anhand eines Ausführungsbeispiels im Zusammenhang mit den Zeichnungen beschrieben, in denen:

Fig. 1 eine schematische Ansicht eines Längenmessgerätes zeigt,

Fig. 2 eine Explosionsdarstellung von Teilen des Längenmessgerätes im Bereich des Messschiebers zeigt,

Fig. 3 eine Querschnittsansicht des Längenmessgerätes im Bereich von innerem Schieber und Messschieber zeigt, wobei die Schieberkomponenten selbst nicht dargestellt sind, sondern lediglich die in ihnen angeordneten Permanentmagnete,

Fig. 4 eine schematische Ansicht des Längenmessgerätes im Längsschnitt zeigt,

Fig. 5 die Anregung und das Nachschwingen des Ultraschallwandlers bei Aussenden eines Ultraschallsignals zeigt,

Fig. 6 die digitalisierten Signale des Ultraschallwandlers im Verlaufe seiner Anregung zur Aussendung eins Ultraschallsignals und darauf folgende Aufnahme eines Reflexionssignals als Funktion der Zeit zeigt,

Fig. 7 die Kreuzkorrelation des Anregungssignals mit dem aufgenommenen reflektierten Signal als Funktion der zeitlichen Verschiebung zeigt,

Fig. 8 eine schematische Querschnittsansicht einer alternativen Ausführungsform eines Längenmessgerätes zeigt,

Fig. 9 ein Beispiel für ein anregendes Signal für einen Lautsprecher zur Erzeugung eines akustischen Schallsignals zeigt,

Fig. 10 eine Kreuzkorrelation des ausgesendeten akustischen Schallsignals mit dem vom Mikrophon aufgenommenen reflektierten Schallsignal bei einer ersten Position des inneren Schiebers zeigt, und

Fig. 11 eine Kreuzkorrelation des ausgesendeten akustischen Schallsignals mit den vom Mikrophon aufgenommenen reflektierten Schallsignalen bei einer zweiten Position des inneren Schiebers zeigt.

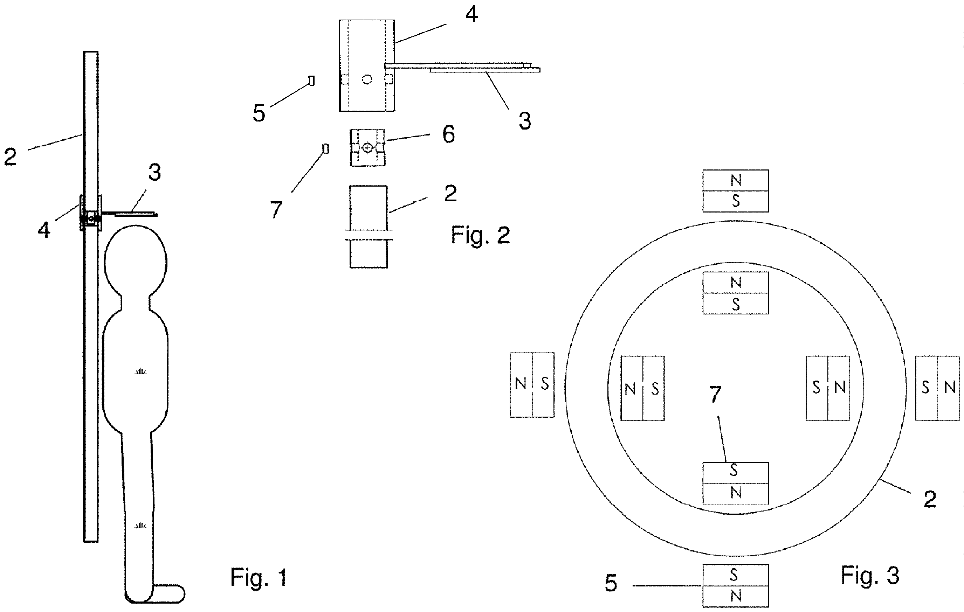

Fig. 1 zeigt eine Seitenansicht eines Längenmessgerätes, das zum Beispiel an einer Wand befestigt sein kann. Das Längenmessgerät weist ein Hohlprofil 2 als lineare Führung auf, auf der außen verschiebbar ein Messschieber 4 gelagert ist, der eine Kopfplatte 3 trägt. Der Messschieber 4 wird soweit abgesenkt, bis die Kopfplatte 3 oben auf dem Kopf der zu messenden Person aufliegt.

In dem Hohlprofil 2, das in diesem Ausführungsbeispiel im Querschnitt kreisringförmig ist, ist ein innerer Schieber 6 verschiebbar gelagert. Der innere Schieber 6 ist im Querschnitt ebenfalls kreisringförmig, so dass der innere Schieber 6 eine zentrale Durchgangsöffnung 16 hat. Die äußeren Abmessungen des inneren Schiebers 6 sind an die inneren Abmessungen des Hohlprofils 2 angepasst, so dass der innere Schieber 6 ohne Spiel, aber gleitfähig im Innenraum des Hohlprofils 2 sitzt. Entsprechend sind die inneren Abmessungen des Messschiebers 4 an die äußeren Abmessungen des Hohlprofils 2 angepasst, so dass der Messschieber 4 ohne Spiel, aber gleitfähig auf dem äußeren Umfang des Hohlprofils 2 gelagert ist.

Fig. 3 zeigt einen Querschnitt durch das Hohlprofil 2 im Bereich des Messschiebers und des inneren Schiebers, wobei die Messschieber- und die innere Schieberkomponente selbst nicht dargestellt sind, sondern nur die Magnetanordnung aus mehreren Permanentmagneten, die in dem inneren Schieber und dem Messschieber eingebracht sind. In dem inneren Schieber 6 sind um den Umfang herum verteilt vier Permanentmagnete 7 eingebracht, die in einem Abstand von 90° zueinander um den Umfang verteilt sind. Entsprechend sind in dem Messschieber 4 ebenfalls vier Permanentmagnete 5 eingebracht, die in entsprechenden Abständen von 90° um den äußeren Messschieber verteilt angeordnet sind. Dabei ist die Anordnung der Permanentmagnete so, dass einander gegenüberliegende Permanentmagnete 5 und 7 des Messschiebers 4 und des inneren Schiebers 6 mit entgegengesetzten Polen aufeinander zu ausgerichtet sind. In dem dargestellten Ausführungsbeispiel wird dies dadurch erreicht, dass die Permanentmagnete 7 des inneren Schiebers mit einem magnetischen Pol, in diesem Beispiel mit dem Nordpol, nach außen ausgerichtet sind, während die Permanentmagnete 5 ebenfalls mit diesem Magnetpol, hier dem Nordpol, nach außen gerichtet angeordnet sind, so dass sich jeweils ein Paar von Permanentmagneten 5 und 7 mit entgegengesetzten Polen aufeinander zu gerichtet gegenüberliegt. Auf diese Weise sind der Messschieber 4 und der innere Schieber 6 magnetisch miteinander gekoppelt. Dadurch folgt der innere Schieber 6 jeder Bewegung des Messschiebers 4 entlang des Hohlprofils 2. In Fig. 2 ist jeweils nur einer der Permanentmagnete 5 und 7 gezeigt.

Grundsätzlich können natürlich auch mehr oder weniger als vier Permanentmagnete pro Schieberkomponente vorgesehen sein, z.B. nur jeweils ein Permanentmagnet in dem Messschieber 4 und dem inneren Schieber 6. Es ist sogar möglich, das überhaupt nur ein Magnet entweder in dem inneren Schieber 6 oder in dem Messschieber 4 vorgesehen ist und die andere Schieberkomponente ohne eigenen Magneten ferro- oder paramagnetisches Material enthält, so dass eine magnetische Anziehung zwischen dem inneren Schieber 6 und dem Messschieber 4 bewirkt wird. Der oder die Magnete der Magnetanordnung sind vorzugsweise Permanentmagnete, grundsätzlich sind aber auch Elektromagnete einsetzbar.

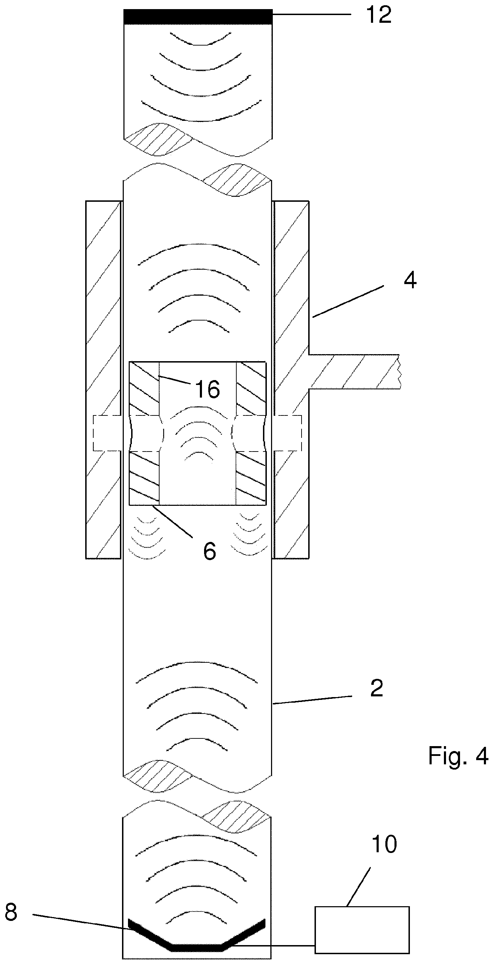

Fig. 4 zeigt eine schematische Schnittansicht des Längenmessgerätes zur Erläuterung seiner Funktionsweise. An einem Ende des Hohlprofils, in diesem Beispiel am unteren Ende, ist im Inneren des Hohlprofils 2 ein Ultraschallwandler 8 angeordnet. Dieser steht in Verbindung mit einer Steuer- und Auswerteeinheit 10, die vorteilhaft auch innerhalb des Hohlprofils 2 oder, wie aus Gründen der einfacheren Darstellbarkeit dargestellt, außerhalb des Hohlprofils angeordnet sein kann. Die Steuer- und Auswerteeinheit 10 erzeugt Anregungssignale für den Ultraschallwandler 8, der daraufhin ein Ultraschallsignal aussendet, das sich in dem Hohlprofil 2 nach oben fortpflanzt. Der innere Schieber 6 hat einen unteren Wandbereich, der durch die untere Stirnfläche des kreisringförmigen Schiebers gebildet wird. An diesem unteren Wandbereich wird ein Teil des ausgesendeten Ultraschallsignals reflektiert und läuft zurück zu dem Ultraschallsignal 8. Durch die Messung der Laufzeit vom Aussenden des Ultraschallsignals vom Ultraschallwandler 8 bis zum Auffangen der vom unteren Wandbereich des inneren Schiebers 6 reflektierten Signale, kann der Abstand zwischen Ultraschallwandler 8 und dem unteren Wandbereich des inneren Schiebers 6 berechnet werden, wobei auf die Einzelheiten der Berechnung der Laufzeit weiter unten eingegangen wird.

Ein Teil des von dem Ultraschallwandler 8 abgestrahlten Ultraschallsignals durchläuft den inneren Schieber 6 durch dessen zentrale Durchgangsöffnung 16 und pflanzt sich weiter in dem Hohlprofil fort. In dem dargestellten Ausführungsbeispiel ist das Hohlprofil an der gegenüberliegenden Seite durch eine Wand 12 verschlossen. Der den inneren Schieber 6 passierende Teil des Ultraschallsignals wird schließlich an der Wand 12 reflektiert und durchläuft wiederum die Durchgangsöffnung 16 des inneren Schiebers zurück zum Ultraschallwandler 8. Auch für diesen Teil des Ultraschallsignals kann die Laufzeit bestimmt werden. Da der absolute Abstand von dem Ultraschallwandler 8 zu der Wand 12 des Hohlprofils bekannt ist, kann dadurch eine Kalibration der Abstandsbestimmung aus der Laufzeitmessung durchgeführt werden.

Grundsätzlich kann für eine solche Kalibration auch ein anderes reflektiertes Signal verwendet werden; z.B. wird auch am oberen Ende der Durchgangsöffnung 16 ein Teil des Ultraschallsignals reflektiert. Aus der Laufzeitdifferenz zwischen dem am unteren Wandbereich des inneren Schiebers 6 und dem am oberen Ende der Durchgangsöffnung 16 des inneren Schiebers reflektierten Ultraschallsignal kann bei bekannter axialer Länge des inneren Schiebers 6 in Längsrichtung des Hohlprofils eine Kalibration der Abstandsbestimmung aus der Laufzeit durchgeführt werden. Um die Reflexion am oberen Rand der Durchgangsöffnung 16 zu verstärken, kann an diesem oberen Rand auch ein nach innen vorstehender Wandbereich vorgesehen sein, so dass am oberen Ende der Durchgangsöffnung 16 eine nach innen gerichtete Schulter ausgebildet ist. Es können grundsätzlich auch andere Störstellen im Inneren des Hohlprofils vorgesehen sein, die Reflexionen eines von dem Ultraschallwandler 8 abgestrahlten Ultraschallsignals erzeugen und aus deren Laufzeiten bei bekannten Positionen entlang des Hohlprofils wiederum eine Kalibration der Abstandsbestimmung aus der Laufzeitmessung vorgenommen werden kann.

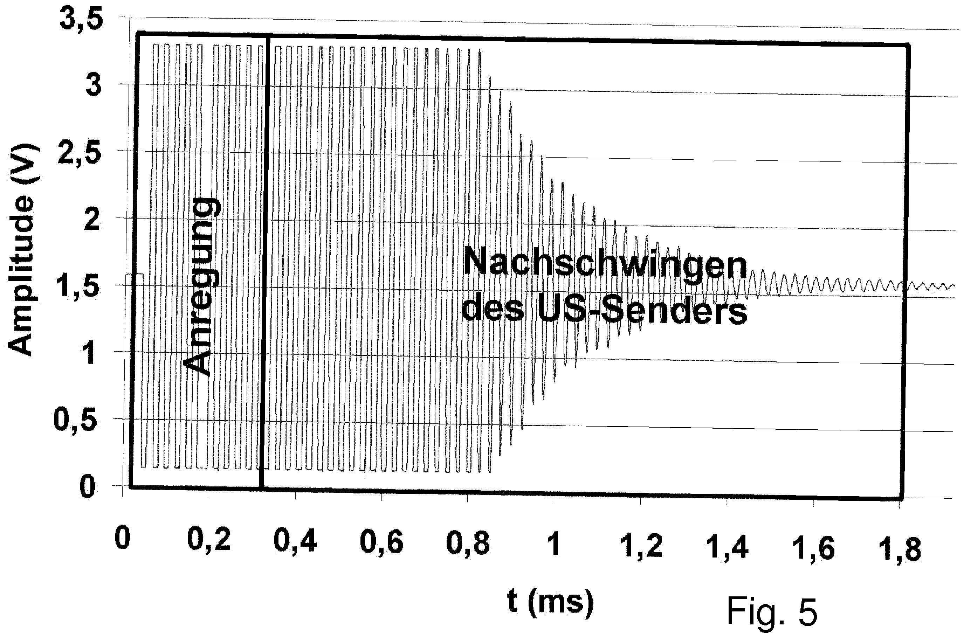

Fig. 5 zeigt die Amplitude der Anregung des Ultraschallwandlers 8 und das nachfolgende Nachschwingen des Ultraschallwandlers als Funktion der Zeit. Die Steuer- und Auswerteeinheit 10 regt den Ultraschallwandler 8 mit fünf Pulsen mit einer Periodendauer von 25 µs (1/40 kHz) an, wonach ein Phasensprung um 180° und danach fünf weitere Pulse gleicher Pulslänge folgen. Der Phasensprung um 180° zeigt sich in dem vergrößerten Abstand zwischen dem fünften und sechsten Puls der Pulsfolge. Der Ultraschallwandler schwingt dann noch eine gewisse Zeit wie dargestellt nach.

Zur Berechnung der Entfernung wird ein charakteristischer Punkt des ausgesandten Ultraschallsignals herangezogen, der sich auch in dem reflektierten Signal zeigen muss. Ein solcher charakteristischer Punkt kann zum Beispiel ein Phasensprung, ein Amplitudensprung oder ein Frequenzsprung sein. Das nach Aussenden des Ultraschallsignals vom Ultraschallwandler aufgenommene Signal wird mit einer im Vergleich zur Ultraschallfrequenz hohen Abtastrate von zum Beispiel $f_S$ = 500 kHz abgetastet und digitalisiert. Findet man den Abtastpunkt des aufgenommenen Reflexionssignals, bei dem sich der charakteristische Punkt des ausgestrahlten Signals wiederfindet, so kann daraus die Laufzeit berechnet werden. Wenn man die Nummer des Abtastpunktes, bei dem sich der charakteristische Punkt im Reflexionssignal wiederfindet, ab dem Abtastpunkt, bei dem der charakteristische Punkt im ausgesendeten Signal auftritt, mit $N_S$ bezeichnet, so ergibt sich daraus für den Abstand L des Reflexionspunktes:

$$L = \frac{N_S c_l}{2 f_s}$$

Hierbei ist $N_S$ die Nummer des Abtastpunktes, bei dem der charakteristische Punkt im reflektierten Signal auftrat, gezählt ab dem Abtastpunkt des charakteristischen Punktes des ausgesendeten Signals, $c_1$ die Schallgeschwindigkeit und $f_s$ die Abtastfrequenz, zum Beispiel $f_s$ = 500 kHz.

Bei einer solchen Messung wird die theoretische Auflösung durch die Abtastfrequenz bestimmt und ergibt für $f_s$ = 500 kHz:

$$\Delta L = \frac{c_l}{2 f_s} \approx 0{,}343 \, mm$$

Eine solche Genauigkeit ist für die Längenmessung zur Bestimmung der Körpergröße von Personen akzeptabel.

Um den charakteristischen Punkt des ausgesandten Signals im reflektierten Signal aufzufinden, kann zum Beispiel eine Kreuzkorrelation berechnet werden. Im Falle von abgetasteten digitalisierten Signalen kann die Kreuzkorrelation für diskrete Systeme wie folgt beschrieben werden:

$$F(n) = \sum_{l=1}^{M} S(l+n) \cdot W(l)$$

S ist das digitalisierte vom Ultraschallwandler empfangene Signal und W ist eine dem ausgesendeten Signal entsprechende digitalisierte Funktion, M ist eine vorbestimmte Zahl von Abtastpunkten, die einer Fensterlänge entspricht. Die Kreuzkorrelation F wird dann am größten, wenn die Verschiebung der das ausgesendete Signal charakterisierenden Funktion gerade genau zu dem reflektierten Signal führt, so dass der charakteristische Punkt des ausgesandten Signals mit dem charakteristischen Punkt des reflektierten Signals zusammenfällt.

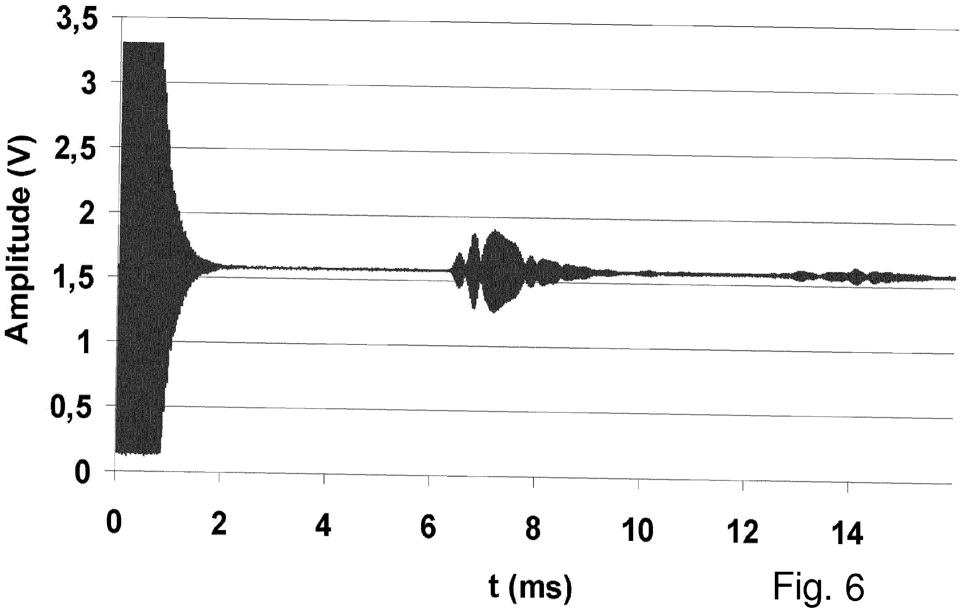

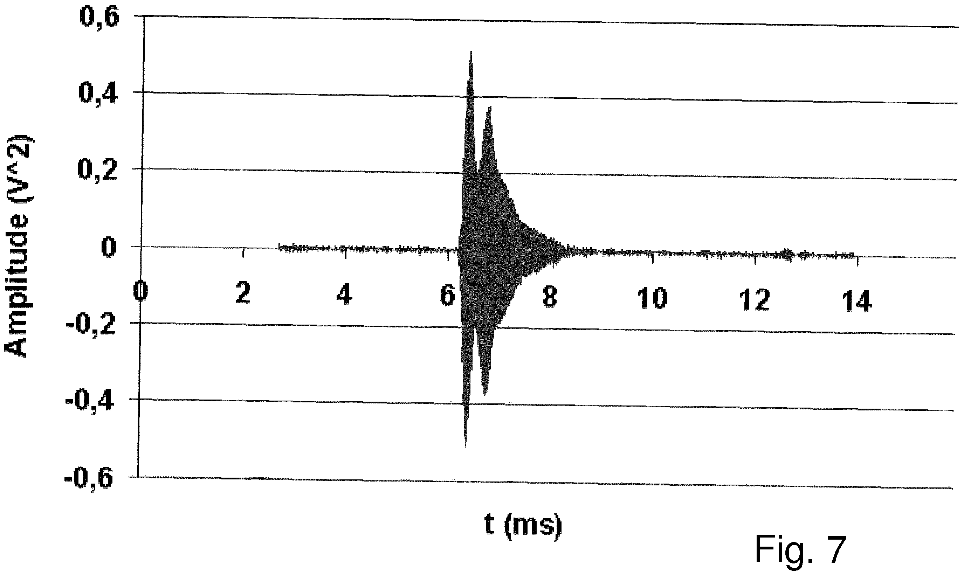

Fig. 6 zeigt die Ausgabe des Ultraschallwandlers als Funktion der Zeit nach einer Anregung wie in Fig. 5 dargestellt. Nach der Anregung und dem Nachschwingen des Ultraschallwandlers werden zunächst für eine Zeit bis etwa 6 ms keine Reflexionen aufgenommen. Im Zeitraum von etwa 6 bis 10 ms ist die Ausgabeamplitude des Ultraschallwandlers durch die erste Reflexion erhöht. In Fig. 7 ist die Kreuzkorrelation als Funktion der zeitlichen Verschiebung zwischen ausgesendetem Signal und reflektierten Signal dargestellt. Die Kreuzkorrelation erreicht ihr Maximum bei etwa 6,2 ms. Dieses Maximum der Kreuzkorrelation wird zur Bestimmung der Laufzeit der Reflexion herangezogen.

In diesem Fall tritt diese Reflexion am unteren Wandbereich des inneren Schiebers auf. In derselben Weise kann eine weitere Reflexion, zum Beispiel vom oberen Ende des Hohlprofils aufgenommen werden und wiederum durch Bilden der Kreuzkorrelation die Laufzeit für diese weitere Reflexion bestimmt werden. Da der Abstand des Ultraschallwandlers zu dem Punkt der weiteren Reflexion oder der Abstand zu der ersten Reflexion bekannt ist, kann durch eine solche Referenzmessung die Abstandsmessung als Funktion der Laufzeit absolut kalibriert werden.

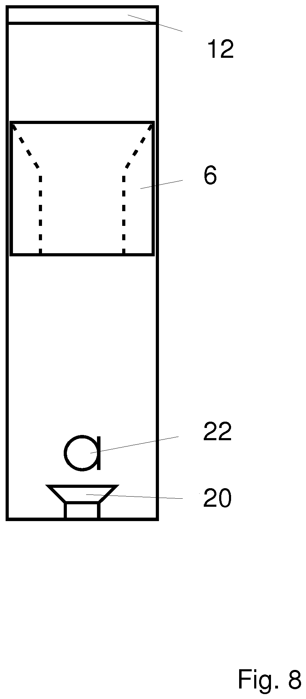

Fig. 8 zeigt eine schematische Querschnittsdarstellung einer alternativen Ausführungsform eines Längenmessgerätes, wobei hier zur Vereinfachung der Messschieber außen am Hohlprofil nicht dargestellt ist. Im Inneren des Hohlprofils befindet sich an dessen unterem Ende ein Lautsprecher 20, der zur Erzeugung eines akustischen Schallsignals geeignet ist. Unter einem akustischen Schallsignal wird in der vorliegenden Anmeldung ein Signal mit Schallwellenlängen im hörbaren Bereich verstanden.

Das akustische Schallsignal, zu dem der Lautsprecher 20 von der Steuer- und Auswerteeinheit angeregt wird, stellt einen kurzen Schallimpuls dar, der sich praktisch wie ein kurzes "Knacken" anhört. Durch die Echos, die durch Reflexionen am unteren Endbereich des inneren Schiebers und zum Beispiel an der Endwand des Hohlprofils erzeugt werden, lassen sich die Laufzeiten der Schallwellen bis zum unteren Ende des Messschiebers und die Laufzeit vom Lautsprecher zur Endwand und zurück zum Mikrophon ermitteln, wodurch sich die Position des inneren Schiebers entlang der Längsachse des Hohlprofils zusammen mit einer Referenzmessung zur Kalibration ableiten lässt.

Die Steuer- und Auswerteeinheit, die in Fig. 8 zur Vereinfachung nicht dargestellt ist, ist dazu eingerichtet, den Lautsprecher 20 so anzusteuern, dass er einen kurzen Impuls eines akustischen Schallsignals sendet. Dieser Impuls breitet sich im Inneren des Hohlprofils aus und wird von einem Mikrophon 22 erfasst. Der Impuls breitet sich weiter aus und trifft auf den unteren Rand des inneren Schiebers 6, der einen Teil des akustischen Schallsignals reflektiert. Der reflektierte Anteil des Impulses läuft dann wieder zurück und trifft auf das Mikrophon 22. Der innere Schieber 6 hat wiederum einen Durchgang 16, der es einem Teil des Impulses des akustischen Schallsignals ermöglicht, den inneren Schieber 6 zu passieren. Dieser Anteil breitet sich dann weiter in dem Hohlprofil 2 aus, bis er auf die Endwand 12 trifft und daran reflektiert wird, woraufhin ein Teil wiederum den Durchgang 16 des inneren Schiebers 6 passiert und schließlich wieder das Mikrophon 22 erreicht. Die Distanz zwischen dem Mikrophon 22 und der Endwand 12 ist konstant und bekannt, so dass durch die Messung der Laufzeit des an der Endwand 12 reflektierten Signals eine absolute Kalibration der Entfernungsmessung möglich ist.

Der Durchgang 16 durch den inneren Schieber 6 ist in diesem Ausführungsbeispiel so gestaltet, dass an dem von dem Lautsprecher 20 abgewandten Ende des Durchgangs 16 möglichst wenig Schall reflektiert wird, um Verluste zu vermeiden und eine maximale Reflexion an der Endwand 12 zu erhalten. In einer alternativen Ausführungsform kann der Durchgang 16 aber auch wie in dem ersten Ausführungsbeispiel so gestaltet sein, dass eine zweite Reflexion am vom Lautsprecher 20 abgewandten Ende des Durchgangs 16 stattfindet, so dass jeweils ein Doppelsignal von an dem inneren Schieber 6 reflektieren Schallwellen erfasst wird, wobei der Abstand der Signale in diesem Doppelsignal zu der bekannten axialen Länge des inneren Schiebers 6 in Beziehung gesetzt werden kann, um eine absolute Kalibration der Entfernungsbestimmung zu erhalten.

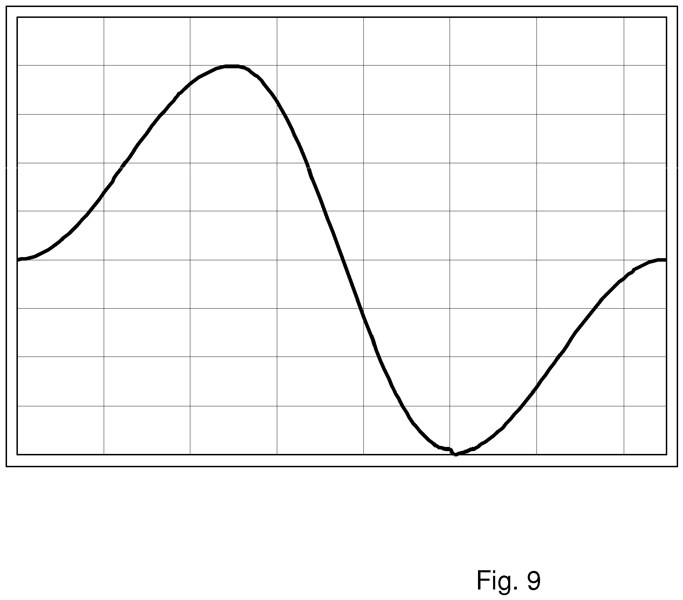

In Fig. 9 ist ein Beispiel für ein anregendes Signal für den Lautsprecher als Funktion der Zeit dargestellt. Das Signal ist so gewählt, dass der Lautsprecher stetig anläuft und wieder heruntergefahren wird; d.h. die dargestellte positive und negative Halbwelle ist nicht sinusförmig, sondern so modifiziert, dass die Steigung zu Beginn der positiven Halbwelle und am Ende der negativen Halbwelle Null ist. Würde der Lautsprecher zu Beginn abrupt hochgefahren oder am Ende abrupt gestoppt, würden Oberwellen angeregt werden, die nicht erwünscht sind. Die Impulsdauer des Signals in Fig. 9 beträgt 0,375 ms.

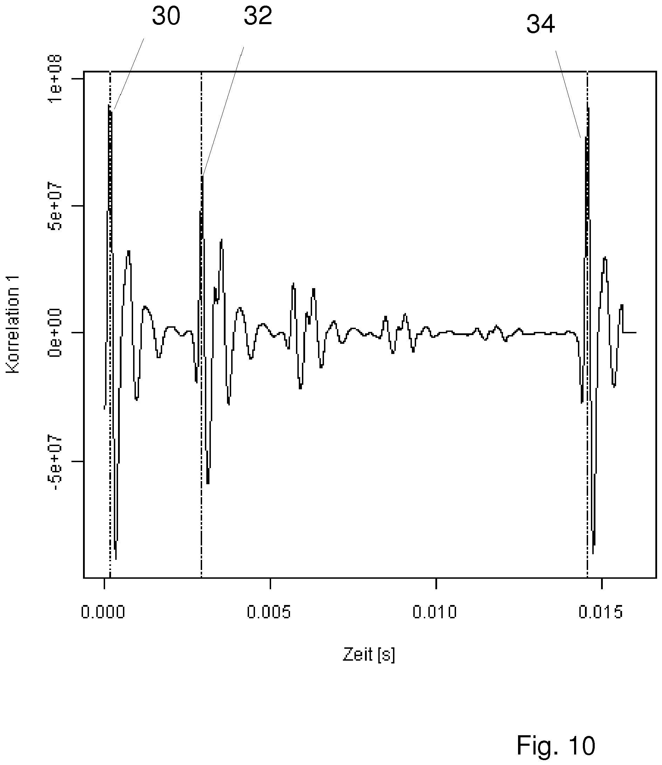

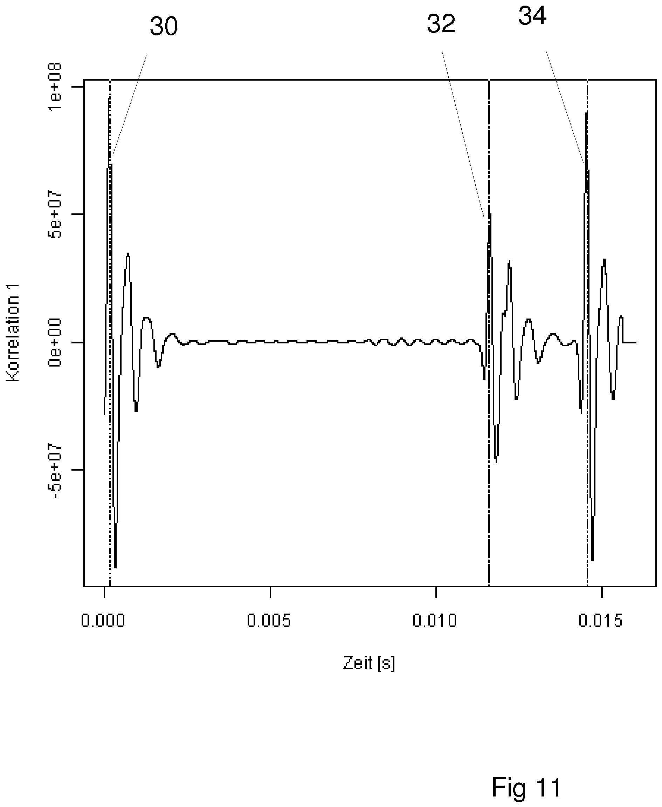

Zwischen dem anregenden Signal und dem mit dem Mikrophon aufgezeichneten Signal wird in diesem Beispiel eine Kreuzkorrelation gebildet. Dies liefert eine Kreuzkorrelation wie in den Fig. 10 und 11 dargestellt.

In Fig. 10 befindet sich der innere Schieber dabei relativ nahe am Mikrophon. In dieser Situation tritt ein erstes Maximum 30 der Kreuzkorrelation auf, das den Abstand zwischen Lautsprecher 20 und Mikrophon 22 widerspiegelt. Das zweite Maximum 32 der Kreuzkorrelation entspricht dem am unteren Wandbereich des inneren Schiebers reflektierten ersten Schallsignal. In dem dargestellten Beispiel befindet sich der Schieber relativ nahe an dem Mikrophon, so dass das an dem inneren Schieber 6 reflektierte erste Schallsignal nach einer relativ kurzen Verzögerungszeit wieder am Mikrophon eintrifft. Das dritte Maximum 34 der Kreuzkorrelation entspricht dem an der Endwand 12 des Hohlprofils reflektierten akustischen Schallsignal, das also die Laufzeit zwischen Lautsprecher 20 zur Endwand 12 und zurück zum Mikrophon 22 ergibt.

Fig. 11 zeigt eine Fig. 10 entsprechende Kreuzkorrelation, wobei in diesem Fall der innere Schieber in größerer Entfernung zu dem Mikrophon 22 liegt, so das die zeitliche Verzögerung bis zum zweiten Maximum 32 der Kreuzkorrelation entsprechend größer ist.

Aus den Kreuzkorrelationen wird die Laufzeit $T_{Mess}$ zwischen dem direkten Signal bei dem ersten Maximum 30 der Kreuzkorrelation und dem ersten Echo entsprechend dem zweiten Maximum 32 ermittelt, das von der Reflexion des akustische Schallsignals am unteren Endwandbereich des inneren Schiebers 6 herrührt. Außerdem wird die Laufzeit $T_{Ref}$ zwischen dem direkten Signal beim ersten Maximum 30 der Kreuzkorrelation und dem Echo der Endwand 12 ermittelt, dass dem dritten Maximum 34 der Kreuzkorrelation entspricht. Für die Abstände gilt dann

$$D_{Mess} = \frac{T_{Mess}}{2} \cdot C$$

$$D_{Ref} = \frac{T_{Ref}}{2} \cdot C$$

wobei C die Schallgeschwindigkeit, $D_{Mess}$ der Abstand zwischen Mikrophon und innerem Schieber und $D_{Ref}$ der Abstand zwischen Mikrophon und Endwand 12 ist.

Damit folgt für den gesuchten Abstand:

$$D_{Mess} = \frac{T_{Mess}}{T_{Ref}} \cdot D_{Ref}$$

Bezugszeichenliste

2    Hohlprofil
3    Kopfplatte
4    Messschieber
5    Permanentmagneten im Messschieber
6    Innerer Schieber
7    Permanentmagnete im inneren Schieber
8    Ultraschallwandler
10   Steuer- und Auswerteeinheit
12   Endwand
20   Lautsprecher
22   Mikrophon
30   Akustisches Schallsignal
32   Reflektiertes Signal des inneren Schiebers
34   Reflektiertes Signal der Endwand

**Patentansprüche**

1. Längenmessgerät zur Messung der Körperlänge einer Person mit einem Messschieber (4), einer linearen Führung in Form eines Hohlprofils (2), an dem der Messschieber außen verschiebbar gelagert ist, um ihn in Auflage auf den Kopf der zu messenden Person bringen zu können, einem innerer Schieber (6), der im Inneren des Hohlprofils verschiebbar gelagert ist und mit dem Messschieber gekoppelt ist, so dass der innere Schieber jeder Bewegung des Messschiebers entlang des Hohlprofils folgt, einer Messeinrichtung zur Messung der Position des inneren Schiebers entlang des Hohlprofils und einer im Außenraum des Hohlprofils sichtbaren Anzeige der durch die Messeinrichtung ermittelten Länge nach Maßgabe der gemessenen Position des inneren Schiebers, **dadurch gekennzeichnet, dass** eine Magnetanordnung (5,7) den Messschieber und den inneren Schieber magnetisch koppelt und dass die Messeinrichtung eine Schallquelle (8; 20), einen Schallempfänger (8; 22) und eine damit verbundene Steuer- und Auswerteeinheit (10) aufweist, die dazu eingerichtet ist, die Schallquelle (8; 20) zur Aussendung eines Schallsignals anzuregen und Ausgabesignale des Schallempfängers (8; 22) auszuwerten, um aus einem an dem inneren Schieber (6) reflektierten ersten Schallsignal dessen Laufzeit zu bestimmen und daraus die Position des inneren Schiebers entlang des Hohlprofils zu berechnen, wobei die Steuer- und Auswerteeinheit (10) weiter dazu eingerichtet ist, in dem Ausgabesignal des Schallempfängers (8; 22) ein weiteres reflektiertes Schallsignal, das an einem bekannten Punkt entlang des Hohlprofils oder in bekannter Entfernung zum Reflexionsort des ersten reflektierten Schallsignals reflektiert worden ist, zu erfassen und dessen Laufzeit als Referenzmessung in die Berechnung der Position des inneren Schiebers eingehen zu lassen.

2. Längenmessgerät nach Anspruch 1, **dadurch gekennzeichnet, dass** die Schallquelle (8; 20) und Schallempfänger (8; 22) in einem Endbereich des Hohlprofils (2) angeordnet ist und dass die Steuer- und Auswerteeinheit (10) dazu eingerichtet ist, die Laufzeit eines Schallsignals zu bestimmen, das von einem der Schallquelle zugewandten unteren Wandbereich des inneren Schiebers (6) reflektiert wurde, und dass der innere Schieber einen ringförmigen Aufbau mit einer Durchgangsöffnung (16) in axialer Richtung hat, wobei die Steuer- und Auswerteeinheit (10) dazu eingerichtet ist, als weiteres reflektiertes Schallsignal ein an dem von der Schalquelle (8; 20) abgewandten Ende der Durchgangsöffnung reflektiertes und zu dem Schallempfänger (8; 22) zurückgekehrtes Schallsignal zu erfassen.

3. Längenmessgerät nach Anspruch 1, **dadurch gekennzeichnet, dass** die Schallquelle (8; 20) in einem Endbereich des Hohlprofils (2) angeordnet ist und dass die Steuer- und Auswerteeinheit (10) dazu eingerichtet ist, die Laufzeit eines Schallsignals zu bestimmen, das von einem der Schallquelle zugewandten unteren Wandbereich des inneren Schiebers reflektiert wurde, dass der innere Schieber (6) einen ringförmigen Aufbau mit einer Durchgangsöffnung (16) in axialer Richtung hat, und dass das Hohlprofil (2) an dem der Schallquelle gegenüberliegenden Ende durch eine Endwand (12) geschlossen ist, wobei die Steuer- und Auswerteeinheit dazu eingerichtet ist, als weiteres reflektiertes Signal ein von der Schallquelle (8; 20) ausgesandtes Schallsignal zu erfassen, nachdem es die Durchgangsöffnung (16) des inneren Schiebers (6) passiert und an der Endwand (12) an dem gegenüberliegenden geschlossenen Ende des Hohlprofils reflektiert worden und zu dem Schallempfänger (8; 22) zurückgekehrt ist.

4. Längenmessgerät nach einem der vorhergehenden Ansprüche, **dadurch gekennzeichnet, dass** in dem Hohlprofil an vorbestimmten Positionen Störstellen angeordnet sind, die Reflexionen von Schallsignalen erzeugen, und dass die Steuer- und Auswerteeinheit dazu eingerichtet ist, die an den Störstellen reflektierte Schallsignale als weitere reflektierte Signale im Ausgabesignal des Schallempfängers zu erfassen und deren Laufzeiten als Referenzmessungen in die Berechnung der Position des inneren Schiebers eingehen zu lassen.

5. Längenmessgerät nach einem der vorhergehenden Ansprüche, **dadurch gekennzeichnet, dass** das Hohlprofil (2) vollkommen geschlossen ist, so dass das Innere des Hohlprofils (2) gegenüber der Umgebung abgeschlossen ist.

6. Längenmessgerät nach einem der vorhergehenden Ansprüche, **dadurch gekennzeichnet, dass** die Steuer- und Auswerteeinheit dazu eingerichtet ist, die Entfernungsbestimmung über ein TOF-Verfahren (Time of Flight) durchzuführen.

7. Längenmessgerät nach einem der vorhergehenden Ansprüche, **dadurch gekennzeichnet, dass** die Steuer- und Auswerteeinheit (10) dazu eingerichtet ist, die Schallquelle mit einem periodischen Anregungssignal anzuregen, dem eine charakteristische Signaleigenschaft wie ein Phasensprung, ein Amplitudensprung oder ein Frequenzsprung aufgeprägt ist, und durch Bilden einer Korrelation der vom Schallempfänger aufgenommenen reflektierten Signalen mit dem Anregungssignal die Laufzeit zu berechnen, insbesondre eine Kreuzkorrelation zu berechnen und die Laufzeit beim Maximum der Kreuzkorrelation zu bestimmen.

8. Längenmessgerät nach einem der vorhergehenden Ansprüche, **dadurch gekennzeichnet, dass** die Magnetanordnung (5, 7) wenigstens einen Permanentmagneten an dem Messschieber (4) und einen Permanentmagneten an dem inneren Schieber (6) aufweist, die so angeordnet sind, dass entgegengesetzte Pole der beiden Permanentmagnete aufeinander zu weisend zueinander ausgerichtet sind.

9. Längenmessgerät nach Anspruch 8, **dadurch gekennzeichnet, dass** jeweils vier Permanentmagnete (5, 7) an dem inneren Schieber (6) und an dem Messschieber (4) so zueinander angeordnet sind, dass jeweils ein Paar eines Permanentmagneten an dem Messschieber und an dem inneren Schieber mit entgegengesetzten Polen aufeinander zu weisend zueinander ausgerichtet sind.

10. Längenmessgerät nach einem der Ansprüche 1 bis 7, **dadurch gekennzeichnet, dass** die Magnetanordnung einen Permanentmagneten an einem von Messschieber und innerem Schieber aufweist und dass der andere von Messschieber und innerem Schieber ferro- oder paramagnetisches Material enthält.

11. Längenmessgerät nach einem der vorhergehenden Ansprüche, **dadurch gekennzeichnet, dass** der Innendurchmesser des Hohlprofils (2) so ausgewählt ist und sind die Schallquelle und die Steuer- und Auswerteeinheit dazu eingerichtet, dass die Frequenzen der Schallsignals von der Steuer- und Auswerteeinheit (10) so vorgegeben werden, dass der Innendurchmesser des Hohlprofils kleiner als die halbe Wellenlänge des Schallsignals ist.

12. Längenmessgerät nach einem der vorhergehenden Ansprüche, **dadurch gekennzeichnet, dass** die Schallquelle eine Ultraschallquelle und der Schallempfänger eine Ultraschallempfänger ist, wobei vorzugsweise Ultraschallquelle und Ultraschallempfänger durch einen einheitlichen Ultraschallwandler (8) in Form eines Transceivers gebildet sind.

13. Längenmessgerät nach einem der Ansprüche 1 bis 11, **dadurch gekennzeichnet, dass** die Schallquelle einen Lautsprecher (20) zur Erzeugung eines akustischen Schallsignals und der Schallempfänger ein Mikrophon (22) zum Aufnehmen akustischer Schallsignale aufweist.

## Claims

1. A length measuring device for measuring the physical length of a person, said length measuring device having a measuring slide (4), a linear guide in the form of a hollow profile (2), on which the measuring slide is mounted so it is externally displaceable, to be able to bring it into contact on the head of the person to be measured, an inner slide (6), which is mounted so it is displaceable in the interior of the hollow profile, and which is coupled to the measuring slide so that the inner slide follows every movement of the measuring slide along the hollow profile, a measuring unit for measuring the position of the inner slide along the hollow profile, and a display, which is visible in the exterior of the hollow profile, of the length ascertained by the measuring unit in accordance with the measured position of the inner slide, **characterized in that** a magnet assembly (5, 7) magnetically couples the measuring slide to the inner slide, and that the measuring unit has a sound source (8; 20), a sound receiver (8; 22), and a control and analysis unit (10) connected thereto, which is configured to excite the sound source (8; 20) to emit a sound signal and to analyze output signals of the sound receiver (8; 22), to determine a runtime of a first sound signal reflected on the inner slide (6) and to calculate the position of the inner slide along the hollow profile therefrom, wherein the control and analysis unit (10) is furthermore configured to capture a further reflected sound signal in the output signal of the sound receiver (8; 22), which has been reflected at a known point along the hollow profile or at a known distance to the reflection location of the first reflected sound signal, and to cause its runtime to be incorporated as a reference measurement in the calculation of the position of the inner slide.

2. The length measuring device as claimed in claim 1, **characterized in that** the sound source (8; 20) and sound receiver (8; 22) are arranged in an end region of the hollow profile (2), and the control and analysis unit (10) is configured to determine the runtime of a sound signal, which was reflected from a lower wall region of the inner slide (6) facing toward the sound source, and the inner slide has a ring-shaped structure having a passage opening (16) in the axial direction, wherein the control and analysis unit (10) is configured to register a sound signal, which is reflected on the end of the passage opening facing away from the sound source (8; 20) and returns to the sound receiver (8; 22), as a further reflected sound signal.

3. The length measuring device as claimed in claim 1, **characterized in that** the sound source (8; 20) is arranged in an end region of the hollow profile (2), and the control and analysis unit (10) is configured to determine the runtime of a sound signal, which was reflected from a lower wall region of the inner slide facing toward the sound source, the inner slide (6) has a ring-shaped structure having a passage opening (16) in the axial direction, and the hollow profile (2) is closed on the end opposite the sound source by an end wall (12), wherein the control and analysis unit is configured to register a sound signal emitted from the sound source (8; 20) as a further reflected signal, after it has passed the passage opening (16) of the inner slide (6) and has been reflected on the end wall (12) on the opposing closed end of the hollow profile and has returned to the sound receiver (8; 22) .

4. The length measuring device as claimed in any one of the preceding claims, **characterized in that** discontinuities are arranged in the hollow profile at predetermined positions, which generate reflections of sound signals, and the control and analysis unit is configured to capture the sound signals reflected at the discontinuities as further reflected signals in the output signal of the sound receiver and to have the runtimes thereof incorporated as reference measurements in the calculation of the position of the inner slide.

5. The length measuring device as claimed in any one of the preceding claims, **characterized in that** the hollow profile (2) is completely closed, so that the interior of the hollow profile (2) is closed off in relation to the environment.

6. The length measuring device as claimed in any one of the preceding claims, **characterized in that** the control and analysis unit is configured to carry out the distance determination via a TOF method (time of flight).

7. The length measuring device as claimed in any one of the preceding claims, **characterized in that** the control and analysis unit (10) is configured to excite the sound source using a periodic excitation signal, to which a characteristic signal property such as a phase jump, an amplitude jump, or a frequency jump is superimposed, and to calculate the runtime by forming a correlation of the reflected signals recorded by the sound receiver with the excitation signal, in particular to calculate a cross-correlation and to determine the runtime at the maximum of the cross-correlation.

8. The length measuring device as claimed in any one of the preceding claims, **characterized in that** the magnet assembly (5, 7) has at least one permanent magnet on the measuring slide (4) and one permanent magnet on the inner slide (6), which are arranged so that opposing poles of the two permanent magnets are aligned in relation to one another facing toward one another.

9. The length measuring device as claimed in claim 8, **characterized in that** four permanent magnets (5, 7) in each case on the inner slide (6) and on the measuring slide (4) are arranged in relation to one another such that in each case one pair of a permanent magnet on the measuring slide and on the inner slide are aligned in relation to one another having opposing poles facing toward one another.

10. The length measuring device as claimed in any one of claims 1 to 7, **characterized in that** the magnet assembly has one permanent magnet on one of measuring slide and inner slide, and the other of measuring slide and inner slide contains ferromagnetic material or paramagnetic material.

11. The length measuring device as claimed in any one of the preceding claims, **characterized in that** the internal diameter of the hollow profile (2) is selected and the sound source and the control and analysis unit are configured such that the frequencies of the sound signal are predefined by the control and analysis unit (10) such that the internal diameter of the hollow profile is less than half of the wavelength of the sound signal.

12. The length measuring device as claimed in any one of the preceding claims, **characterized in that** the sound source is an ultrasound source and the sound receiver is an ultrasound receiver, wherein preferably ultrasound source and ultrasound receiver are formed by a unified ultrasonic transducer (8) in the form of a transceiver.

13. The length measuring device as claimed in any one of claims 1 to 11, **characterized in that** the sound source has a loudspeaker (20) for generating an acoustic sound signal and the sound receiver has a microphone (22) for recording acoustic sound signals.

## Revendications

1. Appareil de mesure de longueur pour mesurer la longueur du corps d'une personne avec un pied à coulisse (4), avec un guide linéaire sous la forme d'un profilé creux (2) sur lequel le pied à coulisse est logé à l'extérieur de manière à pouvoir coulisser afin de pouvoir être mis en appui sur la tête de la personne à mesurer, avec un coulisseau intérieur (6) qui est logé de manière à pouvoir coulisser à l'intérieur du profilé creux et qui est couplé au pied à coulisse de telle sorte que le coulisseau intérieur suit chaque mouvement du pied à coulisse le long du profilé creux, avec un dispositif de mesure pour mesurer la position du coulisseau intérieur le long du profilé creux et avec un affichage, visible à l'extérieur du profilé creux, de la longueur déterminée par le dispositif de mesure d'après la position mesurée du coulisseau intérieur,
**caractérisé en ce qu'**un dispositif magnétique (5, 7) couple magnétiquement le pied à coulisse et le coulisseau intérieur
et **en ce que** le dispositif de mesure comporte une source sonore (8 ; 20), un récepteur sonore (8 ; 22) et une unité de commande et d'évaluation (10) qui est reliée à ceux-ci et qui est conçue pour activer la source sonore (8 ; 20) en vue de l'émission d'un signal sonore et pour évaluer des signaux de sortie du récepteur sonore (8 ; 22) dans le but de déterminer, à partir d'un premier signal sonore réfléchi au niveau du coulisseau interne (6), le temps de propagation de celui-ci et de calculer à partir de là la position du coulisseau interne le long du profilé creux, dans lequel l'unité de commande et d'évaluation (10) est en outre conçue pour détecter dans le signal de sortie du récepteur sonore (8 ; 22) un autre signal sonore réfléchi qui a été réfléchi au niveau d'un point connu le long du profilé creux ou à une distance connue par rapport au point de réflexion du premier signal sonore réfléchi et d'introduire le temps de propagation dudit signal comme mesure de référence dans le calcul de la position du coulisseau intérieur.

2. Appareil de mesure de longueur selon la revendication 1, **caractérisé en ce que** la source sonore (8 ; 20) et le récepteur sonore (8 ; 22) sont agencés dans une zone d'extrémité du profilé creux (2)
et **en ce que** l'unité de commande et d'évaluation (10) est conçue pour déterminer le temps de propagation d'un signal sonore qui a été réfléchi par une zone de paroi intérieure, proche de la source sonore, du coulisseau intérieur (6),
et **en ce que** le coulisseau intérieur a dans la direction axiale une structure annulaire avec une ouverture de passage (16),
dans lequel l'unité de commande et d'évaluation (10) est conçue pour détecter comme autre signal sonore réfléchi un signal sonore réfléchi au niveau de l'extrémité, éloignée de la source sonore (8 ; 20), de l'ouverture de passage et renvoyé au récepteur sonore (8 ; 22).

3. Appareil de mesure de longueur selon la revendication 1, **caractérisé en ce que** la source sonore (8 ; 20) est agencée dans une zone d'extrémité du profilé creux (2)

et **en ce que** l'unité de commande et d'évaluation (10) est conçue pour déterminer le temps de propagation d'un signal sonore qui a été réfléchi par une zone de paroi inférieure, proche de la source sonore, du coulisseau intérieur,

**en ce que** le coulisseau intérieur (6) a dans la direction axiale une structure annulaire avec une ouverture de passage (16),

et **en ce que** le profilé creux (2) est fermé par une paroi d'extrémité (12) au niveau de l'extrémité située à l'opposé de la source sonore,

dans lequel l'unité de commande et d'évaluation est conçue pour détecter comme autre signal réfléchi un signal sonore émis par la source sonore (8 ; 20) après qu'il est passé par l'ouverture de passage (16) du coulisseau intérieur (6) et qu'il a été réfléchi par la paroi d'extrémité (12) au niveau de l'extrémité fermée opposée du profilé creux et a été renvoyé au récepteur sonore (8 ; 22).

4. Appareil de mesure de longueur selon l'une quelconque des revendications précédentes, **caractérisé**

**en ce que** sont agencés dans le profilé creux, dans des positions prédéterminées, des points parasites qui produisent des réflexions de signaux sonores et **en ce que** l'unité de commande et d'évaluation est conçue pour détecter les signaux sonores réfléchis par les points parasites comme autres signaux réfléchis dans le signal de sortie du récepteur sonore et pour introduire leurs temps de propagation comme mesures de référence dans le calcul de la position du coulisseau intérieur.

5. Appareil de mesure de longueur selon l'une quelconque des revendications précédentes, **caractérisé en ce que** le profilé creux (2) est complètement fermé de telle sorte que l'intérieur du profilé creux (2) est fermé par rapport à l'environnement.

6. Appareil de mesure de longueur selon l'une quelconque des revendications précédentes, **caractérisé en ce que** l'unité de commande et d'évaluation est conçue pour effectuer une détermination de distance par le biais d'un procédé TOF (Time of Flight).

7. Appareil de mesure de longueur selon l'une quelconque des revendications précédentes, **caractérisé en ce que** l'unité de commande et d'évaluation (10) est conçue pour activer la source sonore avec un signal d'activation périodique qui est marqué par une propriété de signal caractéristique telle qu'un saut de phase, un saut d'amplitude ou un saut de fréquence et pour calculer le temps de propagation en formant une corrélation des signaux réfléchis reçus par le récepteur sonore avec le signal d'activation, en particulier pour calculer une corrélation croisée et déterminer le temps de propagation au maximum de la corrélation croisée.

8. Appareil de mesure de longueur selon l'une quelconque des revendications précédentes, **caractérisé en ce que** le dispositif magnétique (5, 7) comporte au moins un aimant permanent au niveau du pied à coulisse (4) et un aimant permanent au niveau du coulisseau intérieur (6), lesquels aimants permanents sont agencés de telle manière que des pôles opposés des deux aimants permanents sont orientés l'un par rapport à l'autre de manière à être dirigés l'un vers l'autre.

9. Appareil de mesure de longueur selon la revendication 8, **caractérisé en ce que**, à chaque fois, quatre aimants permanents (5, 7) sont agencés de telle manière les uns par rapport aux autres au niveau du coulisseau intérieur (6) et au niveau du pied à coulisse (4) que, à chaque fois, une paire d'un aimant permanent au niveau du pied à coulisse et au niveau du coulisseau intérieur se présente avec des pôles opposés orientés l'un par rapport à l'autre de manière à être dirigés l'un vers l'autre.

10. Appareil de mesure de longueur selon l'une quelconque des revendications 1 à 7, **caractérisé en ce que** le dispositif magnétique comporte un aimant permanent au niveau d'un des éléments pied à coulisse ou coulisseau intérieur et **en ce que** l'autre des éléments pied à coulisse ou coulisseau intérieur contient un matériau ferromagnétique ou paramagnétique.

11. Appareil de mesure de longueur selon l'une quelconque des revendications précédentes, **caractérisé en ce que** le diamètre intérieur du profilé creux (2) est choisi de telle sorte et la source sonore et l'unité de commande et d'évaluation sont conçues de telle sorte que les fréquences des signaux sonores sont prescrites par l'unité de commande et d'évaluation (10) de telle sorte que le diamètre intérieur du profilé creux est inférieur à la moitié de la longueur d'onde du signal sonore.

12. Appareil de mesure de longueur selon l'une quelconque des revendications précédentes, **caractérisé en ce que** la source sonore est une source d'ultrasons et le récepteur sonore est un récepteur d'ultrasons, la source d'ultrasons et le récepteur d'ultrasons étant formés de préférence par un transducteur d'ultrasons (8) unique sous la forme d'un émetteur-récepteur.

13. Appareil de mesure de longueur selon l'une quelconque des revendications 1 à 11, **caractérisé en ce que** la source sonore comporte un haut-parleur (20) destiné à produire un signal sonore acoustique

**EP 2 925 225 B1**

et le récepteur sonore comporte un microphone (22) destiné à recevoir des signaux sonores acoustiques.

Fig. 3

Fig. 2

Fig. 1

Fig. 4

Fig. 5

Fig. 6

Fig. 7

Fig. 8

Fig. 9

Fig. 10

Fig 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9817974 A1
- DE 3428132 A1
- JP H02239843 A


**In der Beschreibung aufgeführte Nicht-Patentliteratur**

- **S. S. HUANG et al.** A high accuracy ultrasonic distance measurement system using binary frequency shift-keyed signal and phase detection. *Review of Scientific Instruments,* Oktober 2002, vol. 73 (10), 3671-3677
- **R. QUEIROS et al.** A new method for high resolution ultrasonic ranging in air. *XVIII Imeko World Congress, Metrology for a Sustainable Development,* 17. September 2006
- **M. M. SAAD et al.** *IEEE Transactions on Instrumentation and Measurement,* Oktober 2011, vol. 60 (10), 3334-3341